# FASCICULE DE BREVET EUROPEEN

(11) **EP 2 879 731 B1**
(45) Date de publication et mention de la délivrance du brevet: **05.10.2016**
(21) Numéro de dépôt: 13756580.0
(22) Date de dépôt: 19.07.2013
(51) Int. Cl.: A61L 31/12, A61L 31/14

(54) **DISPOSITIF RESORBABLE ET RADIO-OPAQUE POUR LA FIXATION OSSEUSE**
RESORBIERBARE UND RÖNTGENDICHTE VORRICHTUNG ZUR KNOCHENFIXIERUNG
RESORBABLE AND RADIOPAQUE DEVICE FOR BONE FIXATION

(30) Priorité: 02.08.2012 FR 1257514
(43) Date de publication de la demande: 10.06.2015
(73) Titulaire: Teknimed, 65500 Vic-en-Bigorre (FR)
(72) Inventeur: LEONARD, Alain, 207 Nosy BE (MG); LEONARD, Carole, F-31380 Paulhac (FR); SENDER, Cyril, F-31500 Toulouse (FR); LIGNON, Olivier, F-81500 Ambres (FR); HALBIN, Gautier, F-31470 Fontenilles (FR); SAHRAOUI, Nouredine, F-31100 Toulouse (FR)
(74) Mandataire: Argyma
(86) Numéro de dépôt international: PCT/FR2013/051749
(87) Numéro de publication internationale: WO 2014/020259

(56) Documents cités:
- WO-A1-2005/009496
- WO-A2-03/059409
- WO-A2-2007/140325
- US-A1- 2006 121 084
- US-A1- 2010 316 591

## Description

La présente invention appartient au domaine des matériels chirurgicaux, et se rapporte plus particulièrement à des systèmes de fixation utilisés dans la réparation osseuse.

Elle a pour objet un dispositif d'ostéosynthèse à la fois résorbable et radio-opaque, réalisé à l'aide d'un mélange composite de matériaux à dégradation progressive comprenant au moins un composé polymère ou copolymère et une charge minérale apportée par une céramique.

L'ostéosynthèse regroupe l'ensemble des procédés qui permettent de maintenir en place deux structures osseuses suite à une fracture, une arthrodèse ou une ostéotomie. On y a recours par exemple quand la réduction (c'est-à-dire la remise des extrémités osseuses face à face) ne peut pas se faire par des manoeuvres extérieures ou lorsque les deux fragments ne sont pas stables. Elle met en oeuvre divers matériels permettant de maintenir entre eux les deux fragments de l'os (ou un os et un implant) : plaques, tiges, clous, vis, broches, agrafes, etc. Le but est d'arriver à la consolidation de l'os en position anatomique.

L'ostéosynthèse par plaque et vis est utilisée en chirurgie osseuse depuis des dizaines d'années. Les plaques peuvent être de formes variées (droite, longue, courbée, en X, etc...) en fonction de leur emplacement, et sont maintenues par des vis de différents diamètres et de différentes longueurs. Ces matériels sont réalisés avec des matériaux tolérés par l'organisme, d'autant qu'ils ne sont pas toujours retirés après la consolidation de l'os.

L'ostéosynthèse par plaque et vis est considérée comme un moyen sûr pour obtenir une bonne consolidation osseuse, quand on peut la réaliser parfaitement. Pour cela, un certain nombre de conditions doivent être respectées, ou du moins recherchées. Il est tout d'abord indispensable d'assurer un maintien rigide. L'épaisseur de la plaque doit être calculée pour résister aux efforts appliqués au segment considéré en fonction des capacités de résistance du matériau constitutif de la plaque utilisée. Par exemple dans le cas d'une fracture de l'humérus, la fixation va se faire par une plaque métallique épaisse (par exemple 3 mm). Une seconde condition est que la plaque doit être étroitement solidaire de l'os, les vis devant rester en place malgré les vibrations ou autres sollicitations pouvant créer un jeu et conduire à leur descellement. Dans l'exemple susmentionné, on fixera la plaque à l'os avec trois à quatre vis bicorticales de part et d'autre du foyer de fracture, car les contraintes auxquelles l'humérus est soumis sont importantes. Bien entendu, les caractéristiques des vis (forme, taille) sont choisies en relations avec celles de la plaque avec laquelle elles coopèrent.

Le matériau utilisé va aussi jouer un rôle important. Les dispositifs plaques et vis proposés aux chirurgiens ont été pendant longtemps en métal, généralement en titane ou en acier inoxydable. Ces métaux ou alliages métalliques, bien que parfaitement tolérés par l'organisme, présentent deux inconvénients. D'une part, la plaque n'étant que rarement retirée (environ 15% des cas), elle va être impliquée dans la durée au fonctionnement cinématique de l'organe qu'elle a permis de ressouder. Or, le fait qu'elle reste en place entraîne que les contraintes mécaniques ne sont pas transmises de façon homogène dans l'os concerné. Il en résulte une fragilisation de l'os au niveau des points de contact entre l'os et la plaque, à savoir essentiellement au niveau des vis. Les fractures secondaires sont donc fréquentes.

L'emploi de systèmes en polymère de synthèse offre une amélioration de ce point de vue du fait de leur plus grande élasticité. Cependant, en revanche ces polymères ont une résistance mécanique plus faible que celle des métaux, mais aussi que celle des os. Leur moindre résistance à la flexion impose une épaisseur plus élevée pour obtenir le même niveau de rigidité que celui de pièces métalliques. Les systèmes plaques et vis sont donc la plupart du temps surdimensionnés par rapport à leur équivalent métallique. Par exemple, une plaque métallique pour le poignet a communément une épaisseur d'environ 1 mm, alors que pour obtenir une résistance équivalente dans la même indication, une plaque réalisée en polymère résorbable devra avoir une épaisseur d'au moins 3 mm.

Dans l'un et l'autre cas, la plaque présente une épaisseur significative qui, bien qu'elle soit réduite au maximum avec les systèmes métalliques, entraîne à moyen et long terme des risques d'irritation des nerfs et/ou des tendons qui passent à proximité. Ceci peut également entraîner à terme une rupture desdits nerfs ou tendons.

Pour remédier à ces inconvénients, il a été proposé une alternative qui est fondée sur l'emploi de matériaux résorbables. On utilise des polymères qui présentent l'avantage d'être totalement résorbables à terme, c'est-à-dire qu'ils disparaissent progressivement de l'organisme, au bout d'un laps de temps qui peut varier d'un polymère particulier à l'autre, mais qui est suffisant pour que l'os ait retrouvé sa solidité. On connaît par exemple les polymères appartenant à la famille des PLA (pour polylactic acids, ou en français acides polylactiques) et des PGA (pour polyglycolic acids, ou acides polyglycoliques en français).

Cependant, l'emploi des polymères, qu'ils soient résorbables ou non, présente d'autres difficultés qui limitent leur utilisation massive en chirurgie.

Un premier problème provient de la stabilité du montage. Les systèmes vis et plaque métalliques actuels les plus performants sont les systèmes verrouillés. Dans ce cas, la vis comporte un premier filet sur le fût qui opère lors de la pénétration et pour le maintien de la vis dans l'os ; et elle comporte un second filet, plus fin que le premier, ménagé au niveau de la tête de vis, qui vient se prendre dans la plaque en fin de course pour verrouiller la vis dans sa position de butée. Or, les systèmes plaque-vis en polymère ne conviennent pas pour ce type de fixation, d'une part parce que les techniques de plasturgie ne permettent pas de fabriquer des pièces en série avec un relief aussi fin et d'autre part, du fait que la résistance relativement faible du matériau ne serait pas suffisante. Ceci est un désavantage significatif des polymères (notamment des polymères résorbables) face au métal.

En second lieu, un problème et non des moindres, provient du suivi post-opératoire, car les polymères sont totalement radio-transparents. Même si cela peut être un avantage dans certains cas (IRM par exemple), de manière générale le fait de ne pas pouvoir visualiser la position du dispositif implanté représente un inconvénient majeur. En effet, les radiographies de contrôle ne laissent apparaître ni la plaque ni les vis, de sorte que le chirurgien ne peut pas savoir où sont exactement situés les matériels qu'il vient de mettre en place, ni vérifier dans la durée l'état de la fixation et de l'évolution vis-à-vis de l'os. Ce contrôle est cependant impératif pour la sécurité à long terme du patient.

Lorsqu'on a recours à des polymères résorbables, s'ajoutent aux inconvénients précédemment évoqués que le chirurgien ne peut pas vérifier la progression de la résorption du système implanté. Or, il peut arriver que les vis se dégradent plus vite que la plaque. Ceci peut aboutir à une migration de la plaque en dehors de sa zone d'implantation et entraîner une inflammation. La surveillance est donc tout aussi indispensable lorsqu'on a recours à un système plaque et vis résorbables.

La confiance du chirurgien vis-à-vis des dispositifs en polymères résorbables n'est donc pas acquise, alors que la disparition progressive du matériel implanté représente un avantage considérable pour le patient, tant durant la phase de consolidation de l'os qu'à long terme après sa guérison.

Chacun des documents US2006/12108, WO2005/009496, US2010/316591, WO2007/140325, WO03/059409 et WO2009/ 120889 décrit des éléments de fixation osseuse (plaque, vis..) constitués d'un matériau composite constitué d'un polymère ou copolymère biorésorbable et d'une charge minérale.

L'objectif de la présente invention est de proposer un système plaque-vis résorbable qui permette de surmonter les problèmes qui viennent d'être énoncés, en conciliant les diverses contraintes identifiées et sans perdre les avantages déjà acquis par les systèmes existants. En particulier, on souhaite bénéficier du caractère résorbable tout en offrant un niveau de sécurité élevé, en assurant une fixation robuste et durable des vis à la plaque et dans l'os durant toute la durée nécessaire à la consolidation osseuse. Un objectif est ainsi de pouvoir réaliser un verrouillage des vis dans la plaque. Un autre objectif est de permettre le contrôle de la position de la plaque et des vis au cours de leur dégradation.. On souhaite en outre obtenir des propriétés mécaniques améliorées, plus proches de celles des structures osseuses, notamment en ce qui concerne le caractère élastique. Pour ce faire, a été mis au point un dispositif plaque et vis réalisé en matériaux totalement résorbables, radio-opaques au moins en partie et dont la résistance mécanique est améliorée.

Plus précisément, le dispositif de fixation osseuse selon l'invention comprend au moins une plaque et un jeu de vis en matériaux résorbables, dans lequel
- ladite plaque est faite d'un premier matériau consistant en un mélange composite comprenant i) un composé polymère ou copolymère résorbable et ii) une charge minérale constituée d'au moins une céramique résorbable,
- et lesdites vis sont faites d'un second matériau consistant en au moins un composé polymère ou copolymère résorbable,
lesdits premier et second matériaux ayant des compositions différentes de sorte que les modules de Young respectifs sont différents.

Par mélange composite, on entend, par similitude avec l'expression utilisée en dentisterie, un matériau comprenant des charges minérales noyées dans une matrice organique (ou synthétique). De manière originale, il a été trouvé que l'on pouvait associer des matériaux résorbables de nature différente, l'un étant organique, l'autre minéral, pour obtenir un matériel d'ostéosynthèse qui soit à la fois totalement résorbable et radio-opaque, tout en présentant des caractéristiques mécaniques tout à fait satisfaisantes, voire meilleures que certains systèmes actuellement utilisés.

Le caractère résorbable d'un matériau est défini en chirurgie comme la propriété qu'il a de se dégrader progressivement dans l'organisme. En l'occurrence les polymères se dégradent en libérant de l'eau et du dioxyde de carbone que l'organisme sait éliminer sans effet négatif. Les céramiques quant à elles libèrent du calcium, du phosphate et d'autres ions en plus petites quantités, qui sont pour l'essentiel intégrés à l'os voisin. On rencontre aussi parfois les termes biorésorbable ou biodégradable, pour signifier que la dégradation se produit dans un milieu biologique. Les vitesses de dégradation sont variables en fonction de la taille de l'objet et du matériau lui-même. L'homme du métier connaît de nombreux matériaux utilisés dans des applications thérapeutiques, disparaissant progressivement au contact de l'eau de l'organisme dans des intervalles de temps plus ou moins longs, compatibles notamment avec la consolidation osseuse, et qu'il qualifie de résorbables.

Les céramiques résorbables sont actuellement utilisées de manière courante en chirurgie en tant que matériau de comblement osseux. Elles sont apportées sous différentes formes (pâtes, gels, poudre, ou sous des formes solides en cubes, bâtonnets, coin) pour boucher des lacunes et solidifient en formant une structure poreuse que les cellules osseuses vont coloniser. Elles renforcent l'os tout en servant de support aux nouvelles cellules qui viennent les remplacer au fur et à mesure qu'elles se dégradent en libérant des ions qui participent en retour au remodelage osseux. Ce type de matériau est très cassant et fragile, et n'est pas utilisé pour la fabrication d'implants structuraux.

Le mélange composite ici défini s'est avéré conférer au dispositif de fixation osseuse des propriétés mécaniques différentes de celles des dispositifs résorbables connus. Ceci se manifeste en premier lieu par le fait que les matériaux mis en oeuvre dans la présente invention présentent un module de Young plus élevé que les plaques intégralement en polymères, le module de Young augmentant avec la charge de céramique résorbable. Ce faisant, la capacité de déformation élastique a diminué, c'est-à-dire que le matériau est plus rigide que les polymères pris seuls. Le module d'élasticité, sans être comparable à celui des métaux, est de deux à trois fois supérieur à celui des pièces en polymère, ce qui le rapproche des caractéristiques propres des os. Il est en outre plus ductile, ce qui signifie qu'il peut enregistrer une certaine déformation sans se rompre. Ceci a un intérêt certain pour la solidité des vis.

Les vis réalisées en un second matériau peuvent quant à elles être constituées à base d'un composé polymère ou copolymère résorbable, celui-ci pouvant être pris seul ou en mélange. Quoi qu'il en soit, le premier et le second matériaux sont choisis de façon à ce que leur module de Young affiche une différence d'au moins 0,5 GPa.

De préférence, la différence entre les modules de Young desdits premier et second matériaux est d'au moins 1 GPa.

En particulier, les vis destinées à maintenir la plaque sur les extrémités osseuses à joindre peuvent être simplement en matériau synthétique, bien que dans ce cas, seule la plaque qu'elles maintiennent sera visible par les techniques de radiographie. Elles peuvent aussi être constituées d'un mélange composite différent de celui de la plaque, comprenant une charge minérale, par exemple une céramique résorbable. Ainsi, selon une caractéristique préférée du dispositif objet de la présente de l'invention, les vis sont faites d'un second matériau consistant en un composé polymère ou copolymère résorbable, en mélange composite avec une charge minérale constituée d'au moins une céramique résorbable.

La matrice organique (de la plaque et le cas échéant des vis) peut être constituée d'au moins un polymère simple (formé à partir d'un seul type de monomère), ou bien d'au moins un copolymère (formé à partir de deux types de monomères, voire davantage). On connaît de tels composés, désignés ensemble ci-après "composés synthétiques", qui sont utilisés communément pour fabriquer les plaques et les vis d'ostéosynthèse. Il s'agit essentiellement d'acides polylactiques et polyglycoliques et de leur copolymères.

Ainsi, selon l'invention, si le composé synthétique est un polymère, il peut être avantageusement choisi parmi les acides polylactiques (PLA ou polylactides), les polydioxanones (PDO), les carbonates de polytriméthylène (PTMC), les acides polyglycoliques (PGA ou polyglycolides), les polycaprolactones.

De même, si le composé synthétique est un copolymère, celui-ci peut être formé d'au moins deux monomères choisis parmi les acides lactiques énentiopurs ou racémiques, la dioxanone, le carbonate de triméthylène, le glycolide, la caprolactone.

Le matériau choisi pour la matrice organique peut être également obtenu par une association physique de deux ou même plusieurs polymères ou copolymères. Dans ce cas, les composés synthétiques choisis sont mélangés et forment une masse uniforme. Ainsi, selon un mode de réalisation particulier de l'invention, le matériau résorbable constituant la plaque et les vis peut comprendre deux composés polymères ou copolymères résorbables différents.

Comme déjà indiqué plus haut, le dispositif de fixation osseuse (ou au moins la plaque) comprend une charge minérale, qui va également être résorbable et qui va lui conférer sa radio-opacité. De manière préférée, au moins une céramique résorbable est choisie parmi les phosphates de calcium, les sulfates de calcium, les phosphates de strontium et les sulfates de strontium, ou toute autre charge minérale résorbable connue de l'homme du métier. Ces céramiques sont connues pour leur bio-compatibilité. Elles sont incorporées à la matrice polymère sous forme de poudre.

La charge minérale peut être modulée, en quantité et en nature, pour répondre au cahier des charges des dispositifs particuliers à chaque segment osseux auquel il est destiné. Cette possibilité de modulation peut être mise à profit de manière particulièrement intéressante pour améliorer la solidité de la fixation, en choisissant des compositions différentes pour la plaque et pour les vis, de manière à ce que les modules de Young respectifs soient différents. En effet, une différence de module entre le matériau de la plaque et celui de la vis va permettre de réaliser un meilleur coincement de la vis dans la plaque. La vis est prise en force dans la plaque et peut être considérée comme verrouillée à l'instar des systèmes plaque-vis métalliques verrouillés. Une différence de charge minérale entre vis et plaque de 10 points et plus apporte un verrouillage correct avec un différentiel d'au moins 0,5 GPa entre les modules de Young respectifs des vis et de la plaque. Une différence de plus de 20 points est préférée, avec dans ce cas un différentiel de l'ordre de 1 GPa au moins. On ne dépassera toutefois pas 50 points d'écarts, la pièce contenant le plus fort taux de céramique devenant trop rigide et fragile au-delà de cette valeur.

Ainsi, selon une caractéristique avantageuse du dispositif objet de l'invention, la teneur en céramique de la plaque est supérieure d'au moins 10 points, et de préférence d'au moins 20 points, à la teneur en céramique des vis, dans la limite de 50 points.

De manière intéressante, selon l'invention, la plaque comporte de 10% à 60% de céramique. Elle peut en comporter de préférence de 25% à 35%, en poids rapporté au poids total du mélange composite. Ces proportions sont sélectionnées comme étant celles pour lesquelles les différentes caractéristiques mécaniques sont combinées de manière optimale, en particulier le rapport rigidité/ductilité, pour répondre aux objectifs de la présente invention. L'homme du métier saura les ajuster selon des besoins spécifiques, en fonction de la nature chimique du composé polymérique et des spécifications de la poudre de céramique.

Ce faisant, les vis peuvent comporter de 0% à 30% de céramique. De préférence, la teneur en céramique dans les vis peut être fixée à un niveau allant de 5% à 15%, en poids rapporté au poids total du mélange composite. On a ainsi un mélange composite moins riche en charge minérale : on tient compte de la petite taille de ces pièces, qui se casseraient si un taux de céramique identique à celui de la plaque était mis en oeuvre. Une telle composition confère en outre aux vis une meilleure résistance à la torsion pour supporter le vissage sans dommage.

Deux avantages majeurs supplémentaires reposent sur cette teneur en céramique minorée par rapport à celle des plaques. Le premier est que la différence des modules d'élasticité qui en découle assure un coincement amélioré de la vis dans la plaque, réalisant ainsi une verrouillage efficace.

Le second avantage est que la vitesse de dégradation de la pièce est d'autant plus élevée que la teneur en polymère du mélange composite est réduite.

Ceci ouvre la possibilité d'un contrôle de la vitesse de résorption par une modulation des rapports entre charge minérale et matrice organique dans les différentes pièces du dispositif objet de l'invention. On peut en premier lieu sélectionner les composants permettant d'obtenir une dégradation selon un rythme déterminé et choisi. En second lieu, et de manière particulièrement intéressante, on peut différencier la vitesse de résorption des différentes pièces (vis et plaque). On évitera ainsi qu'au cours de la dégradation d'un système plaque-vis, les vis ne se dégradent plus vite que la plaque, et que la plaque migre de manière totalement importune hors du site d'implantation.

Selon l'invention, on peut remédier à ce genre d'inconvénient en sélectionnant les compositions de sorte que le mélange composite de la plaque se dégrade plus vite que celui des vis. La teneur en céramique peut être un facteur d'ajustement dans ce but, le composite se dégradant d'autant plus vite qu'il contient de céramique. Un autre paramètre peut être commodément mis en oeuvre, à savoir le choix de la céramique elle-même.

Par exemple, le phosphate β-tricalcique ayant une vitesse de dégradation supérieure à celle de l'hydroxyapatite calcostroncique, on utilisera avantageusement le premier dans la plaque, et la seconde dans les vis. Ainsi la plaque va se dégrader plus vite que les vis éliminant ainsi les risques de migration de la plaque hors du site d'implantation. Selon un mode de réalisation particulièrement intéressant, dans le dispositif conforme à l'invention, outre le composé synthétique polymère ou copolymère,
- la charge minérale de ladite plaque est constituée de phosphate β-tricatcique, et
- la charge minérale desdites vis est constituée d'hydroxyapatite calcostroncique.

Enfin, il est recommandé de choisir soigneusement la nature du composé synthétique, car elle est extrêmement importante sur plusieurs plans. En effet, si une plaque métallique est inerte et n'apporte aucun nouveau composant dans le corps humain, une plaque résorbable va au contraire libérer des monomères et des oligomères. Elle peut également se fracturer en petits morceaux entraînant des réactions inflammatoires secondaires. Tout ceci contribue à limiter la confiance des chirurgiens.

C'est pourquoi, selon une caractéristique préférée, la plaque et les vis sont constituées d'un mélange composite comprenant un poly(L-lactide-co-D,L-lactide) 70:30 et une charge minérale céramique. Ces copolymères sont des polyesters aliphatiques thermoplastiques obtenus à partir de l'isomère énentiopur L et du racémique de l'acide lactique en rapport 70 pour 30 en masse, qui ont des propriétés mécaniques voisines de celles du poly(L-lactide) 100, et qui contrairement à ce dernier, présentent l'avantage de rester à l'état amorphe tout au long du processus de dégradation. De ce fait, ils se dégradent couche par couche de l'extérieur vers l'intérieur, en libérant des acides lactiques et glycoliques ainsi que des oligomères assimilables par l'organisme, sans générer de petites particules à l'origine des réactions inflammatoires secondaires.

La présente invention sera mieux comprise grâce à la description qui va être faite de certaines variantes de réalisation, en relation avec les figures annexées, dans lesquelles :
- la fig.1 est une vue en perspective d'un dispositif d'ostéosynthèse comprenant une plaque de radius et une de ses vis.
- la fig. 2 est un diagramme montrant l'évolution du Module de Young (en GPa) en fonction de la charge minérale d'un mélange composite mis en oeuvre dans l'invention.
- la fig. 3 est un diagramme montrant l'évolution de l'élongation maximale avant rupture en fonction de la charge minérale d'un mélange composite mis en oeuvre dans l'invention.

### EXEMPLE 1 : Plaque de radius à vis transparentes

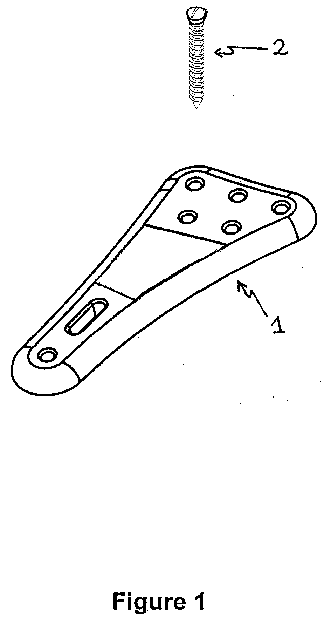

Une plaque 1 antérieure de radius composée d'un mélange composite consistant en 70% de poly(70/30 ;UDL)lactide et 30% de phosphate β-tricalcique a été fabriquée. Le copolymère a été obtenu auprès de EVONIK (RESOMER LR). La céramique utilisée est le phosphate β-tricalcique fabriqué par TEKNIMED. La matrice et la céramique ont été mélangés par voie chimique. La poudre obtenue a été mise en oeuvre par injection moulage. La plaque 1 obtenue est représentée figure 1.

Un jeu de vis 2 résorbables mais non opaques en poly(70/30 ;UDL)lactide (RESOMER LR fourni par EVONIK) a été mis en forme par injection moulage. Une de ces vis 2 est représentée figure 1.

Les modules de Young des deux matériaux ont été déterminés à 5,4 GPa pour le mélange composite de la plaque contre 3,2 GPa pour le matériau polymère des vis, soit un différentiel de 2,2 GPa. Des simulations sur modèles osseux synthétiques ont été effectuées qui ont mis en évidence que le verrouillage est excellent. Le mode de rupture est en effet la casse de la plaque, et non le déverrouillage des vis.

Le dispositif a été testé par implantation sur le radius de neuf moutons et son évolution a été observée pendant 1 an. La plaque montre une parfaite biointégration (aptitude d'un matériau à être colonisé par les cellules vivantes) à 3 mois, 6 mois et 1 an sur tous les animaux. On constate en outre qu'après un an d'implantation, les plaques sont considérablement plus dégradées que les vis, voire dans un cas, la plaque était totalement résorbée.

On observe également que les vis sont restées totalement solidaires de la plaque dans laquelle elles sont insérées : le verrouillage des vis sur les plaques est toujours assuré après un an.

### EXEMPLE 2 : Plaque de radius à vis opaques

Une plaque antérieure de radius composée d'un mélange composite consistant en 50% de poly(70/30 ;UDL)lactide et 50% de phosphate β-tricalcique a été fabriquée. Le copolymère a été obtenu auprès de EVONIK (RESOMER LR). La céramique utilisée est le phosphate β-tricalcique fabriqué par TEKNIMED. La matrice et la céramique ont été mélangés par voie chimique. La poudre obtenue a été mise en oeuvre par injection moulage, selon la même forme qu'à l'exemple 1 (figure 1).

Un jeu de vis résorbables et opaques composée d'un mélange composite consistant en 90% de poly(70/30; L/DL)lactide et 10% d'hydroxyapatite calcostroncique a été réalisé. Le copolymère est fourni par EVONIK (RESOMER LR). L'hydroxyapatite calcostroncique est fabriquée par TEKNIMED. La matrice et la céramique ont été mélangés par voie chimique. La poudre obtenue a été mise en oeuvre par injection moulage.

### EXEMPLE 3 : Evolution du Module de Young et de l'élongation

Une gamme de mélanges composites a été préparée comprenant un copolymère, en l'occurrence du poly(70/30; L/DL)lactide, et une céramique, en l'occurrence du TCP (phosphate β-tricalcique), à différentes teneurs, selon le même protocole qu'à l'exemple 1.

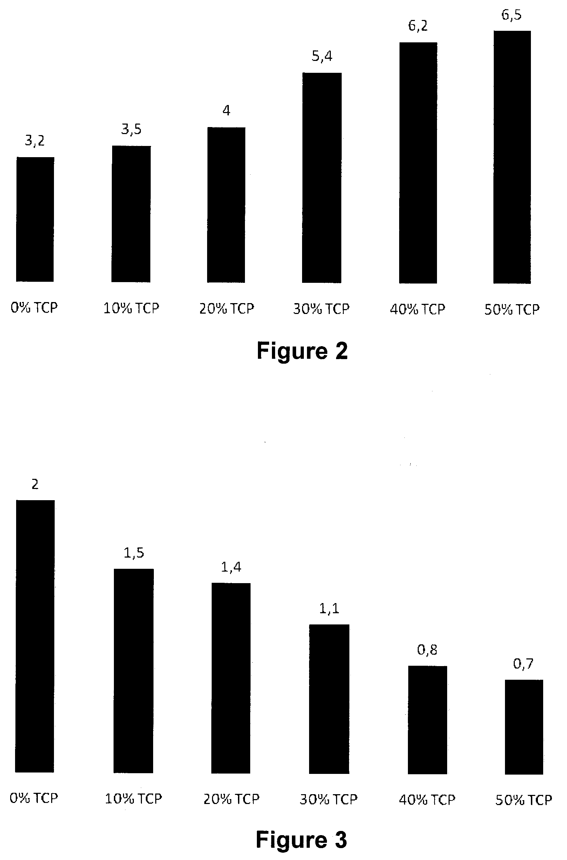

Le diagramme présenté à la figure 2 montre l'évolution du Module d'Young en GPa). Le diagramme présenté à la figure 3 montre l'évolution de l'élongation à la rupture (en %).

Le mélange composite présente un module de Young plus élevé que le matériau 100% polymères, et il augmente avec la charge de céramique résorbable. A l'inverse, l'élongation maximale décroît quand la concentration en céramique augmente. Plus la concentration en céramique augmente plus le matériau devient dur et cassant.

Ceci démontre l'intérêt d'employer conformément à l'invention, un mélange composite plus riches en céramiques pour la fabrication d'une plaque, afin de privilégier la rigidité, mais d'employer mélange composite relativement moins riche en céramique pour la fabrication d'une vis, pour laquelle il est plus important de privilégier la plasticité.

## Revendications

1. Dispositif de fixation osseuse comprenant au moins une plaque et un jeu de vis en matériaux résorbables, **caractérisé en ce que**
- ladite plaque est faite d'un premier matériau consistant en un mélange composite comprenant i) un composé polymère ou copolymère résorbable et ii) une charge minérale comportant au moins une céramique résorbable,
- et lesdites vis sont faites d'un second matériau comportant au moins un composé polymère ou copolymère résorbable,
lesdits premier et second matériaux ayant des compositions différentes de sorte que les modules de Young respectifs sont différents.

2. Dispositif selon la revendication 1, **caractérisé en ce que** la différence entre les modules de Young desdits premier et second matériaux est d'au moins 0,5 GPa.

3. Dispositif selon la revendication 2, **caractérisé en ce que** la différence entre les modules de Young desdits premier et second matériaux est d'au moins 1 GPa.

4. Dispositif selon l'une des revendications précédentes, **caractérisé en ce que** les vis sont faites d'un second matériau comprenant un composé polymère ou copolymère résorbable, en mélange composite avec une charge minérale comportant au moins une céramique résorbable.

5. Dispositif selon l'une des revendications précédentes, **caractérisé en ce que** ledit composé polymère est choisi parmi les acides polylactiques, les polydioxanones, les carbonates de polytriméthylène, les acides polyglycoliques, les polycaprolactones.

6. Dispositif selon l'une des revendications précédentes, **caractérisé en ce que** ledit copolymère est formé d'au moins deux monomères choisis parmi les acides lactiques énantiopurs ou racémiques, le carbonate de triméthylène, le glycolide, la dioxanone, la caprolactone.

7. Dispositif selon l'une des revendications précédentes, **caractérisé en ce que** ladite au moins une céramique résorbable est choisie parmi les phosphates de calcium, les sulfates de calcium, les phosphates de strontium et les sulfates de strontium.

8. Dispositif selon l'une quelconque des revendications précédentes, **caractérisé en ce que** la teneur pondérale en céramique de la plaque est supérieure d'au moins 10 points à la teneur pondérale en céramique des vis, dans la limite de 50 points.

9. Dispositif selon la revendication précédente, **caractérisé en ce que** la teneur pondérale en céramique de la plaque est supérieure d'au moins 20 points, à la teneur pondérale en céramique des vis, dans la limite de 50 points.

10. Dispositif selon l'une des revendications précédentes, **caractérisé en ce que** la plaque comporte de 10% à 60% de céramique, en poids rapporté au poids total du mélange composite.

11. Dispositif selon la revendication précédente, **caractérisé en ce que** la plaque comporte de 25% à 35% de céramique, en poids rapporté au poids total du mélange composite.

12. Dispositif selon l'une des revendications précédentes, **caractérisé en ce que** les vis comportent de 0% à 30% de céramique, en poids rapporté au poids total du mélange composite.

13. Dispositif selon la revendication précédente, **caractérisé en ce que** les vis comportent de 5% à 15% de céramique, en poids rapporté au poids total du mélange composite.

14. Dispositif selon l'une des revendications précédentes, **caractérisé en ce que**
- la charge minérale de ladite plaque est constituée de phosphate β-tricalcique, et
- la charge minérale desdites vis est constituée d'hydroxyapatite calcostroncique.

15. Dispositif selon l'une des revendications précédentes, **caractérisé en ce que** la plaque et les vis sont constituées d'un mélange composite comprenant un poly(L-lactide-co-D,L-lactide) 70:30 et une charge minérale céramique.

## Patentansprüche

1. Vorrichtung zur Knochenfixierung, die mindestens eine Platte und einen Schraubensatz aus resorbierbaren Materialien umfasst, **dadurch gekennzeichnet, dass**
- die Platte aus einem ersten Material hergestellt ist, welches aus einer verbundartigen Mischung besteht, die i) eine resorbierbare Polymer- oder Copolymerverbindung umfasst sowie ii) einen mineralischen Füllstoff, der mindestens einen resorbierbaren Keramikstoff aufweist,
- die Schrauben aus einem zweiten Material hergestellt sind, welches mindestens eine resorbierbare Polymer- oder Copolymerverbindung aufweist,
wobei das erste und das zweite Material unterschiedliche Zusammensetzungen haben, sodass sich ihre Elastizitätsmoduln voneinander unterscheiden.

2. Vorrichtung nach Anspruch 1, **dadurch gekennzeichnet, dass** der Unterschied zwischen den Elastizitätsmoduln des ersten und des zweiten Materials mindestens 0,5 GPa beträgt.

3. Vorrichtung nach Anspruch 2, **dadurch gekennzeichnet, dass** der Unterschied zwischen den Elastizitätsmoduln des ersten und des zweiten Materials mindestens 1 GPa beträgt.

4. Vorrichtung nach einem der vorhergehenden Ansprüche, **dadurch gekennzeichnet, dass** die Schrauben aus einem zweiten Material hergestellt sind, welches eine resorbierbare Polymer- oder Copolymerverbindung umfasst, in verbundartiger Mischung mit einem mineralischen Füllstoff, der mindestens einen resorbierbaren Keramikstoff aufweist.

5. Vorrichtung nach einem der vorhergehenden Ansprüche, **dadurch gekennzeichnet, dass** die Polymerverbindung aus den Polymilchsäuren, den Polydioxanonen, des Polytrimethylencarbonaten, den Polyglycolsäuren, den Polycaprolactonen ausgewählt ist.

6. Vorrichtung nach einem der vorhergehenden Ansprüche, **dadurch gekennzeichnet, dass** das Copolymer aus mindestens zwei Monomeren gebildet ist, die aus den enantiomerenreinen oder racemischen Milchsäuren, Trimethylencarbonat, Glycolid, Dioxanon, Caprolacton ausgewählt sind.

7. Vorrichtung nach einem der vorhergehenden Ansprüche, **dadurch gekennzeichnet, dass** der mindestens eine resorbierbare Keramikstoff aus den Calciumphosphaten, den Calciumsulfaten, den Strontiumphosphaten und den Strontiumsulfaten ausgewählt ist.

8. Vorrichtung nach einem beliebigen der vorhergehenden Ansprüche, **dadurch gekennzeichnet, dass** die Platte einen gewichtsmäßigen Gehalt an Keramikstoff aufweist, der den gewichtsmäßigen Gehalt der Schrauben an Keramikstoff um mindestens 10 Prozentpunkte übertrifft, höchstens jedoch um 50 Prozentpunkte.

9. Vorrichtung nach dem vorhergehenden Anspruch, **dadurch gekennzeichnet, dass** die Platte einen gewichtsmäßigen Gehalt an Keramikstoff aufweist, der den gewichtsmäßigen Gehalt der Schrauben an Keramikstoff um mindestens 20 Prozentpunkte übertrifft, höchstens jedoch um 50 Prozentpunkte.

10. Vorrichtung nach einem der vorhergehenden Ansprüche, **dadurch gekennzeichnet, dass** die Platte 10 % bis 60 % an Keramikstoff aufweist, nach Gewicht unter Bezugnahme auf das Gesamtgewicht der verbundartigen Mischung.

11. Vorrichtung nach dem vorhergehenden Anspruch, **dadurch gekennzeichnet, dass** die Platte 25 % bis 35 % an Keramikstoff aufweist, nach Gewicht unter Bezugnahme auf das Gesamtgewicht der verbundartigen Mischung.

12. Vorrichtung nach einem der vorhergehenden Ansprüche, **dadurch gekennzeichnet, dass** die Schrauben 0 % bis 30 % an Keramikstoff aufweisen, nach Gewicht unter Bezugnahme auf das Gesamtgewicht der verbundartigen Mischung.

13. Vorrichtung nach dem vorhergehenden Anspruch, **dadurch gekennzeichnet, dass** die Schrauben 5 % bis 15 % an Keramikstoff aufweisen, nach Gewicht unter Bezugnahme auf das Gesamtgewicht der verbundartigen Mischung.

14. Vorrichtung nach einem der vorhergehenden Ansprüche, **dadurch gekennzeichnet, dass**
- der mineralische Füllstoff der Platte aus β-Tricalciumphosphat besteht, und
- der mineralische Füllstoff der Schrauben aus Calcium-Strontium-Hydroxyapatit besteht.

15. Vorrichtung nach einem der vorhergehenden Ansprüche, **dadurch gekennzeichnet, dass** die Platte und die Schrauben aus einer verbundartigen Mischung bestehen, die ein Poly(L-lactid-co-D,L-lactid) 70:30 und einen mineralischen Keramikfüllstoff umfasst.

## Claims

1. Bone fixation device comprising at least one plate and a set of screws made of resorbable material, **characterized in that**:
- said plate is made of a first material that consists of a composite mixture comprising i) a resorbable polymer or copolymer compound and ii) an inorganic filler that comprising at least one resorbable ceramic,
- and said screws are made of a second material comprising at least one resorbable polymer or copolymer compound,
said first and second materials having different compositions so that the respective Young's moduli are different.

2. Device according to Claim 1, **characterized in that** the difference between the Young's moduli of said first and second materials is of at least 0,5 Gpa.

3. Device according to Claim 2, **characterized in that** the difference between the Young's moduli of said first and second materials is of at least 1 Gpa.

4. Device according to one of the preceding claims, **characterized in that** the screws are made of a second material comprising one resorbable polymer or copolymer compound, in a composite mixture with an inorganic filler comprising at least one resorbable ceramic.

5. Device according to one of the preceding claims, **characterized in that** said polymer compound is selected from among polylactic acids, polydioxanones, polytrimethylene carbonates, polyglycolic acids, polycaprolactones.

6. Device according to one of the preceding claims, **characterized in that** said copolymer is made of at least two monomers selected from among the enantiopure or racemic lactic acids, trimethylene carbonate, glycolide, dioxanone, caprolactone.

7. Device according to one of the preceding claims, **characterized in that** said at least one resorbable ceramic is selected from among the calcium phosphates, calcium sulfates, strontium phosphates and strontium sulfates.

8. Device according to any of the preceding claims, **characterized in that** the ceramic content by weight of the plate is greater by at least 10 points, than the ceramic content by weight of the screws, not to exceed 50 points.

9. Device according to the preceding claim, **characterized in that** the ceramic content by weight of the plate is greater by at least 20 points, than the ceramic content by weight of the screws, not to exceed 50 points.

10. Device according to one of the preceding claims, **characterized in that** the plate comprises 10% to 60% ceramic, by weight relative to the total weight of the composite mixture.

11. Device according to one of the preceding claims, **characterized in that** the plate comprises 25% to 35% ceramic, by weight relative to the total weight of the composite mixture.

12. Device according to one of the preceding claims, **characterized in that** the screws comprise 0% to 30% ceramic, by weight relative to the total weight of the composite mixture.

13. Device according to one of the preceding claims, **characterized in that** the screws comprise 5% to 15% ceramic, by weight relative to the total weight of the composite mixture.

14. Device according to one of the preceding claims, **characterized in that**
- the inorganic filler of said plate is composed of β-tricalcic phosphate, and
- the inorganic filler of said screws is composed of calcium-strontium hydroxyapatite.

15. Device according to one of the preceding claims, **characterized in that** the plate and the screws are composed of a composite mixture comprising a 70:30 poly(L-lactide-co-D,L-lactide) and a ceramic inorganic filler.
